(19) Europäisches Patentamt | European Patent Office | Office européen des brevets

(11) **EP 4 194 013 A1**

(12) **EUROPEAN PATENT APPLICATION**

(43) Date of publication:
14.06.2023  Bulletin 2023/24

(21) Application number: 21214112.1

(22) Date of filing: 13.12.2021

(51) International Patent Classification (IPC):
$A61L\ 2/10^{(2006.01)}$     $A61L\ 2/16^{(2006.01)}$
$A61L\ 2/18^{(2006.01)}$     $A61L\ 2/26^{(2006.01)}$
$B08B\ 9/30^{(2006.01)}$     $B08B\ 9/42^{(2006.01)}$

(52) Cooperative Patent Classification (CPC):
A61L 2/0047; A23L 3/00; A61L 2/0082; A61L 2/10;
A61L 2/16; A61L 2/18; A61L 2/26; B65G 15/54;
B65G 17/064; B65G 17/083; B65G 21/2045;
B65G 21/2072; B65G 47/22; B65G 47/76;
A61L 2202/14

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: B. Braun Melsungen AG
34212 Melsungen (DE)

(72) Inventors:
• BRADLEY, Paul
  Alnwick, NE66 4TA (GB)
• RODDIS, Alan James
  Chapeltown, S35 2YL (GB)

(74) Representative: Maiwald GmbH
Elisenhof
Elisenstraße 3
80335 München (DE)

(54) **UVC LIGHT DISINFECTION DEVICE**

(57) A disinfection device comprising a conveyor for conveying items to be subjected to disinfection treatment in a forward direction and an irradiation device for irradiating the items to be subjected to disinfection treatment during their transport by the conveyor, wherein the irradiation device comprises at least one radiation source for emitting a UVC radiation. The disinfection device further comprises a guiding device for guiding the items to be subjected to disinfection treatment while being conveyed in a direction that is other than the forward.

Fig. 1

EP 4 194 013 A1

## Description

This invention relates to a disinfection device, in particular for use in aseptic production or processing facilities in the health-products related, biotechnological, pharmaceutical, food and/or nuclear industry sectors, a system comprising a cleanroom and such disinfection device, the use of such disinfection device as a transport connection between a first room and a second room, a method of disinfecting items, and a method of introducing items into a cleanroom.

The largest contributors to the bioburden within a cleanroom are the personnel present and the items passed into the cleanroom, i.e. the materials, tools, containers, samples etc. introduced into the cleanroom. A cleanroom (also "clean room") is a facility that maintains low levels of particulates, such as dust, airborne organisms, vaporised particles, etc. in its interior. A cleanroom may for instance be utilized as a part of an industrial production or scientific research facility, including the manufacture of health products, pharmaceutical products, biotechnological products, semiconductor products, etc. In the context of the present invention, any room or compartment with increased requirements for cleanliness and low biological contamination may be understood to be a cleanroom.

Good gowning techniques using sterile clothing, sterile goggles, sterile gloves, and good cleanroom behaviour can reduce the effect of personnel. This, however, does not solve the problem associated with the items entering the cleanroom.

The use of sterile materials in a Grade B (EudraLex Rules Governing Medicinal Products in the EU, Volume 4: Guidelines to GMP) area is a pre-requisite for aseptic operations. Usually, all goods entering a cleanroom are sterilised by means of, e.g., heat, ethylene oxide or gamma irradiation and are certified sterile. Evidence suggests that it is therefore not these materials or sterilisation methods which usually introduce the bioburden challenge, but the outer packaging that they are presented in, namely bottles, plastic packs, breathable paper packaging, Tyvek packs, etc.

Standard techniques of disinfection of the outer packaging range from manual chemical disinfection techniques to aerosol, vapour, or gaseous disinfection. When manually disinfecting, the inclusion of a sporicidal disinfection stage is recommended as part of a two stage process to transfer materials into a Grade B zone. The efficacy of chemical disinfectants are typically tested against international standards such as the European standard EN 13697. This specifies levels of de-activation of a range of named microorganisms using the chemical disinfectant of 4 Log reduction in 5 minutes, and of 3 Log reduction within 15 minutes (see below for the meaning of "4Log" and "3 Log") for fungal spores of *Aspergillus brasiliensis* and the yeast *Candida albicans.* Local operational standards can vary from country to country. Typically, a manual two stage wipe and spray disinfection process incorporating a sporicidal disinfection stage is employed. Best practice suggests that materials entering goods receipt for onwards disinfection should first be wiped with an additional bactericide, e.g. ethanol, as a decontamination stage to reduce surface bound contamination prior to disinfection. However, this is a manual process that is cumbersome and prone to manual variance.

Standard techniques of disinfection of the outer packaging include manual chemical disinfection techniques with alcohol (isopropanol, ethanol, etc.) and hydrogen peroxide gassing. A guidance document issued in the UK by the NHS in 2015 (Guidance for the Aseptic Processes in the NHS Addressing Sporicidal Issues - Appendix 1) recommends that a sporicidal disinfection stage should be used which operationally has at least a 2 Log reduction within 2 minutes and ideally greater than 3 Log in 2 minutes.

The use of hydrogen peroxide gassing using relatively high concentrations of hydrogen peroxide in the vapour phase, optionally with the addition of peracetic acid, can provide a higher level of bioburden reduction and is effective at eliminating vegetative and sporing microorganisms with an expectation of a 6 Log reduction.

Both manual methods of disinfection and hydrogen peroxide gassing have drawbacks. The manual disinfection process is cumbersome and produces high volumes of waste in the form of wipes and therefore expensive as well as prone to variability through a number of factors, wiping efficacy, quantity of disinfectant applied, environmental humidity levels, operator fatigue, operator compliance and is considered to be at best capable of 3-4 Log reductions if both stages of manual disinfection are applied correctly. Hydrogen peroxide gassing systems are known to be expensive to install, they need specialist equipment with specialist extraction systems, they require structured loading patterns, they use high concentrations of chemicals with high toxicity, and they have varying degrees of success due to the complexity of validations.

UV light was first discovered in 1801 by J. W. Ritter. The germicidal properties of short wavelength light were first published in 1878 by A. Downes et al. (Proceedings of the Royal Society of London, 28 (190-195): 199-212, 1878). Several years later, E. Hertel discovered that it was light in the UVC region that was most effective at killing microorganisms (Zeitschrift für Allgemeine Physiologie, 4, 1-43, 1904 and 5, 95-122, 105). Ultraviolet (UV) light may be used, e.g. for water treatment and disinfection.

For many years, UVC light has been an established method of reducing microorganisms on the surfaces of food such as tomatoes, fruit and vegetables. Unfortunately, short-wave UVC light is harmful to humans as it causes sunburn effects on exposed skin and inflammation of the cornea of the eye, which may lead to temporary or permanent vision impairment. For this reason, the light produced by a germicidal lamp must be carefully shielded against direct viewing, with consideration of reflections and dispersed light.

UV Light is invisible to the human eye. It can be categorised in three distinct bands, A, B and C: UVA with wavelength of 315-400nm, UVB with wavelength of 280-315nm, UVC with wavelength of 100-280nm. Sometimes, UVC is further divided to Far UV (FUV 200-280nm) and Vacuum UV (VUV 100-200nm.

The conventionally used method of generating UVC is using a low pressure mercury or amalgam lamp. These lamps, similar in appearance to fluorescent tubes, contain mercury or a mercury containing amalgam and a starter gas, such as argon at a pressure of, e.g. around 1 Pa. When an electrical charge passes through the lamp, mercury vapour generates UVC light at distinct wavelength peaks, in particular 185 nm and 254 nm.

Soft glass or another material absorbing the peak wavelength of 185 nm may be used as a material for the lamp so that predominantly or exclusively radiation of the peak wavelength of 254 nm is emitted.

Food processing technology and other technologies usually employ low-pressure mercury lamps or similar mercury containing radiation devices.

Mercury lamps and similar devices were set to be banned in many countries from 2020 for environmental reasons because of the toxicity of mercury. However an extension of 5 years was permitted to some mercury containing devices where suitable alternatives were not available.

According to a non-limiting theory, the effectiveness of UVC light is due to its ability to render microorganisms (bacteria, fungi, spores, etc.) inactive; inactivity being the inability to replicate. An experiment performed by the present inventor demonstrated the effectiveness of a commercially available UVC lamp to reduce the formation of colony forming units ("CFU") of bacteria on TSA contact plates. "TSA" is the abbreviation for the growth medium trypticase soy agar. Each plate was contacted on the floor of an unclassified change room prior to daily chemical disinfection. One plate was incubated as normal and the second exposed to 2 minutes of UVC (254nm) light from the UVC lamp, both were incubated under the same conditions. The results were striking in that it was visible to the naked eye that CFU was substantially or even completely supressed by UVC exposure.

According to the non-limiting theory, UVC light is particularly effective in inactivating microorganisms as the proteins, DNA, and RNA absorb the UVC light which in turn causes photoreactions. The UVC induced photoreaction occurs as a result of the absorbance spectra of the molecules such as the DNA bases having maxima, e.g., in the region 260-280nm. According to the non-limiting theory, the excitation caused by the absorption of a photons energy results in a chemical bond between adjacent bases on one DNA strand, or cross linking to another DNA strand and thereby inactivating the DNA. According to the non-limiting theory, the pyrimidines in the DNA are more reactive than the purines so that the most frequent dimers formed are Thymine:Thymine (TT) and to a lesser degree cytosine dimers. In RNA viruses the thymine pyrimidine base is replaced by uracil (U) which may also undergo photoreaction. Additional photochemical reactions may occur in the cells, proteins in the cell have an absorbance peak of, e.g., approximately 280nm.

It has been found out by the present inventor that a factor that may be relevant for effective UVC disinfection of items is effective light exposure. UVC is light and therefore travels in straight lines so the area requiring disinfection must be exposed to the light, wherein any shadowing of the area to be disinfected will reduce the effectiveness of the UVC.

Over the last decade, Light Emitting Diode (LED) technology has developed at a rapid pace, partly to fill the market vacuum anticipated with the obsolescence of mercury-based light but also because the technology uses less energy to create light and is therefore ecologically friendly.

UVC LEDs use semiconductor materials to produce light in a solid-state device. The wavelength of emission may be tunable by adjusting the chemistry of the semiconductor material, giving a selectivity to the emission profile of the LED across, and beyond, the germicidal wavelength band.

The higher the wavelength of the UVC light (e.g., operating in the 260-280 nm range), the better it may be at killing microorganisms or rendering them unable to multiply, and therefore the wider the scope of its application.

Logarithmically (Log) reduction is a mathematical term that is used to express the relative number of living microbes that are eliminated by disinfection. That is, a 1 Log reduction corresponds to inactivating 90 percent of a target microbe with the microbe count being reduced by a factor of 10. For each additional Log number (2 Log, 3 Log, etc.), the disinfection reduces the number of living microbes by a further 90 %. That is, a 2 Log reduction is a 99 % reduction of living microbes, a 3 Log reduction is a 99.9 % reduction of living microbes, etc.

During product efficacy testing, e.g. in microbiology laboratories, the number of colony forming units (CFUs) of the given organism present at the start of the test is counted. Then, the disinfection technique is applied, alongside a control product without disinfection, and the required test time is waited before recounting the number of viable CFUs present.

The result of the difference between the control and the test product is then expressed as a Log reduction. For example, if the number of CFUs in the control was found to be 1,000,000 (or 10E6) and the end result for using the product was only 1,000 (10E3), that would be a Log reduction of 3 or a reduction of 99.9%.

So, if the surface of an item has 1,000,000 test organisms (or CFUs) on it, a 5 Log reduction would reduce the number of microorganisms to ten (99.999% reduction). Similarly a 6 Log reduction reduces the number of microorganisms to one (99.9999% reduction).

According to a non-limiting theory, reducing microorganisms with UVC light is subject to a number of variables including:

- the wattage over a defined surface area (irradiance) of the light source,
- the exposure time during which the disinfected area/item is exposed to the radiation,
- the type of the organism to be inactivated.

The overall light dose (the 'fluence') is the product of the intensity of the light applied (the 'irradiance') and the time it is applied:

$$\text{Irradiance (mW/cm}^2) \times \text{Time (s)} = \text{Fluence (mWs/cm}^2 = \text{mJ/cm}^2)$$

Essentially, the higher the Log reduction requirement of the organism, the greater the dose of the radiation required on the organism is (cf. Bunsen-Roscoe rule). For some organisms the linear relationship does not hold and, e.g., equivalent doses with a great intensity of light can effect a greater Log reduction.

The standard ASTM E315-18 ("Standard Practice for Determining Antimicrobial Efficacy of Ultraviolet Germicidal Irradiation Against Microorganisms on Carriers with Simulated Soil") attempts to establish standard testing methodology. The standard requires bacterial and fungal samples to be prepared using AOAC 961.02 section A(b). This standard test relates to efficacy of spray disinfectants on contaminated nonporous surfaces. This standard specifies for organisms:

- *Salmonella enterica*
- *Staphylococcus aureus*
- *Pseudomonas aeruginosa*
- *Trichophyton mentactrophytes*

The standard BS EN 13697 details criteria of a quantitative surface test for the evaluation of bactericidal or fungicidal activity using chemical disinfectants. Seven organisms are specified:

- *Bacillus subtilus* spores
- *E. coli*
- *Pseudomonas aeruginosa*
- *Aspergillus brasiliensis* spores
- *Candida albicans*
- *Staphylococcus aureus*
- *Enterococcus hirae*

From BS EN 13697, a 4 Log reduction in 5 minutes for bacteria and 3 Log in 15 minutes for fungal spores and yeast is considered acceptable in the case of the application of chemical disinfectants.

For instance, the control of bioburden with an aseptic environment and/or a cleanroom to an acceptable level is a constant challenge in order to reduce the risk of bio-contamination of the products that are produced in the aseptic environment or cleanroom. Well-designed cleanrooms suites with the appropriate design of HVAC system may be able to recover air quality to Grade B levels within a period of 15-20 minutes when the room is no longer occupied. The largest contributors to the bioburden within a cleanroom are not only the personnel present but also the items passed into the room. As discussed above, this aspect still involves a number of difficulties in different areas of application.

According to a non-limiting theory, it may be possible that after imparting damage to the DNA/RNA of organisms rendering them unable to multiply, some organisms, particularly bacteria, can repair part of the damage by exposure to visible light ("photo-reactivation") or without light ("dark repair"). For instance, this could take place by a reverse of the thymine dimer formation by absorbing light in the visible range.

It has been found that the ability to repair may be reduced if the relative humidity is 65% or lower. Moreover, it has been found that the ability to repair may be reduced if a broader UVC wavelength range and/or a higher UVC wavelength is used instead of the 254 nm line generated by a mercury lamp. According to a non-limiting theory this is because of a damage to the cell not related to DNA damage. For example cell protein may have an absorbance peak of 280nm so that comparably high UVC wavelength may more effectively generate nonrepairable cell damage than the UVC line of 254 nm.

Moreover, with an additional treatment with hydrogen peroxide or another chemical agent, an adequate Log reduction may be achieved even if the hydrogen peroxide concentration is much lower than in the case of disinfection techniques using manual hydrogen peroxide spray and impregnated wipes or hydrogen peroxide gassing but no UVC radiation.

As discussed above, the manual disinfection process is cumbersome and prone to variability. Only a rather low Log reduction, such as for instance a 3Log to 4 Log reduction or worse, may be expected. Hydrogen peroxide gassed devices and other gassing devices are known to be expensive to install and difficult to handle. Moreover, high concentrations of chemicals with high toxicity which is not only an environmental issue but also leads to a high risk of accident if a person is unintentionally exposed to the chemicals.

A relatively low initial cost and running cost device and method of bioburden reduction which are operationally robust and/or which reduce chemical exposure and/or which require relatively minimal training, validation, set up, etc. and/or which are not prone to operator variability or fatigue and/or which provide a significant level of bioburden reduction in a short period of time are therefore seen as a significant benefit to the operation of an aseptic unit, cleanroom, operating theatre or other medical facility, etc. Such device and method are also seen as a significant benefit in other situations in which disinfection of items is required.

A device and method which are capable of reducing surface bound viable microorganisms of packaging entering an aseptic unit, a cleanroom, etc. by 4 Log or higher, is deemed particularly advantageous.

Hence, it is an objective of the present invention to provide an improved disinfection device and an improved disinfection method for the disinfection of items. In particular, an effective reduction of surface bound microorganisms present on packaging entering an aseptic unit making use of UVC light unit is desired, e.g. a 4 Log reduction by UVC exposure for approximately 15-20 seconds.

This objective is achieved by the disinfection device according to claim 1, the use according to claim 12, the system according to claim 13, and the methods according to claims 14 and 15. Refinements of the invention are specified in the dependent claims. Any feature set forth in the claims dependent on any independent claim as well as any feature set forth in the description of exemplary embodiments of the invention below can be understood as a feature suitable for refining the claimed disinfection device, the claimed system, the claimed use, the claimed method of disinfection, and the claimed method of inserting items into a cleanroom. That is, for instance, the refinements discussed for the disinfection device are to be understood also as refinements of the disinfection method. If possible from a technical point of view, the features of all described embodiments may be combined with one another.

The disinfection device according to the invention is a disinfection device comprising a conveyor for conveying items to be subjected to disinfection treatment in a forward direction. The disinfection device further comprises an irradiation device for irradiating the items to be subjected to disinfection treatment during their transport by the conveyor. The irradiation device comprises at least one radiation source for emitting a UVC radiation. The disinfection device further comprises a guiding device for guiding the items to be subjected to disinfection treatment while being conveyed in a direction that is other than the forward direction.

Herein, the forward direction is the direction in which an item would move during its transport if no guiding device was present that varies the position of the item relative to the conveyor. The forward direction is hereinafter also referred to as "conveyor direction".

The guiding device has the effect that the item that is conveyed by the conveyor does not only move in the conveyor direction but is also moved relative to the conveyor. The overall movement of the item is therefore a superposition of a movement in conveyor direction and a movement relative to the conveyor, whereby the latter is not in conveyor direction. The latter may, for example, be perpendicular to the conveyor direction, resulting in a total movement that is inclined to the conveyor direction.

The disinfection device according to the invention may for example provide a device associated with relatively low initial cost and relatively low running cost as compared for instance to the manual disinfection method mentioned above. Moreover, with the disinfection device according to the invention, for example an effective bioburden reduction may be achieved without the use of chemical agents or by using relatively low amounts of chemical agents. Moreover, with the disinfection device according to the invention, for example a quicker bioburden reduction may be achieved compared to the manual disinfection method described above. Preferably, a 3Log, 4Log, or higher reduction of microorganism may be achieved. Moreover, the disinfection device according to the invention may for example require less training, validation, set up, etc. and/or may for example be less prone to operator variability or fatigue compared to the manual disinfection method described above. These possible benefits of the disinfection device according to the invention may be particularly advantageous if the device is used, for example, for items entering an aseptic unit, a cleanroom, etc.

It has been found out by the present inventor that effective light exposure in all surface regions of the item to be disinfected may play an important role. In particular, all areas requiring disinfection should be exposed to the UVC radiation, wherein any shadowing of the area to be disinfected has been found to reduce the effectiveness of the UVC radiation.

By applying the guiding device according to the invention, an item that is conveyed by the conveyor is not only moved in the conveyor direction but at the same time in a direction other than the conveyor direction. In other words: The item does not only move in the conveyor direction. In addition, it moves relative to the conveyor.

If, for example, a conveyor with a conveyor belt is used, the item would remain in place on the conveyor belt while being conveyed and would not move relative to the belt if there was no guiding device. The use of the guiding device causes additional movement relative to the belt during conveying. In particular, the item is moved on the surface of the belt.

During irradiation, shadow effects can occur that depend on the position of the item relative to the conveyor. This is particularly the case when at least part of the radiation used for irradiation passes through the conveyor to reach the item. In this case, a conveyor is used which is at least partially transparent to the radiation. Herein, the expression "partially transparent" in particular means that the surface of the conveyor on which the items to be conveyed are positioned has portions with low transparency and portions with high transparency.

If the transparency is not the same in all parts of the conveyor, e.g. transparency is relatively low or even zero over the wire elements of a mesh while transparency is high over open parts of a mesh, a portion of the item located at a point of the belt with a relatively high shadow effect (low or zero transparency) would receive less radiation than a portion of the item located at a point of the belt with a relatively low shadow effect (high transparency). In a portion of the item that receives relatively little radiation, the disinfection effect is relatively low. By moving the item by the guiding device, all parts of the item to be disinfected can be sufficiently irradiated and thus effectively disinfected. An example of a conveyor is a conveyor with a wire mesh conveyor belt. The mesh comprises wires or the like and empty areas between the wires. The underside of an item placed over the wires is not irradiated by a radiation source that is located below the mesh in the portions above the wires or is irradiated less than in the portions above the empty areas because the wires cast a shadow. Because the item is moved relative to the mesh by the guiding device, the position of the item on the conveyor is varied. Thus, the same surface regions of the item are not always located above the wires over time during the conveying, so that all regions can be sufficiently irradiated over time.

Shadow effects that are locally different as well as a locally different radiation effect occurring for other reasons, may not only occur in the case of a partially transparent conveyor and irradiation from below. Locally different shadow effects or a locally different radiation effect may also occur if the radiation is irradiated partially or completely from the side and/or from above onto the items to be disinfected. Local differences can for example result from the fact that radiation sources are not equally effective in all directions. Shadow effects can also result from all items that cast a shadow. Shadow effects can also result from the shape of an item to be disinfected.

Hence, by using the disinfection device according to the invention or by applying the disinfection method according to the invention, an effective disinfection may be possible, e.g. a disinfection that fulfils the requirements defined in the standard BS EN 13697 for chemical disinfectants or an even better disinfection.

Moreover, the disinfection device according to the invention may be used to disinfect large numbers of items in a continuous operating manner with minimal human intervention.

Moreover, the disinfection device according to the invention may be used to disinfect items of various dimensions.

Moreover, the disinfection device according to the invention may be used to disinfect items without the action of expensive sporicidal chemicals or by using only small amounts thereof and without the use of wipes.

Preferably, the guiding device is oriented in a direction that is inclined relative to the conveyor direction both vertically and horizontally.

Additionally or alternatively, the guiding device preferably comprises a nudge wire and/or a nudge bar. A nudge wire (nudge bar) is a wire (bar) that is arranged such that it is in the path of the item to be disinfected during transport by the conveyor, such that the wire (bar) touches the item and guides it sideways causing the item to move additionally relative to the conveyor and thus to perform a movement in the direction according to the orientation of the guide wire (bar). The whole guiding device may be constituted by such a nudge wire or nudge bar or a plurality thereof. The guiding device may in addition comprise further elements.

Additionally or alternatively, the conveyor preferably comprises a conveyor belt partially transparent to the UVC radiation, in particular a mesh conveyor belt, especially a wire mesh conveyor belt, wherein the irradiation device comprises at least one radiation source arranged underneath the conveyor, and wherein the guiding device is configured to move the items to be subjected to disinfection treatment across the conveyor direction, in particular in a sliding movement, on the conveyor belt, Herein, the conveyor belt more preferably comprises a belt body and suspensions protruding from the belt body for suspending the items to be subjected to disinfection above the surface of the belt body. These suspensions may for instance enable suspension of the item to be disinfected above the main body of the wire mesh and allow greater light exposure of the underside of the items to be disinfected.

If a mesh belt is used, it is preferred that the mesh belt comprises mesh openings (i.e. empty areas between the wires or other mesh-forming solid structures) with a total area of at least 50%, in particular at least 75% of the total surface area of the conveyor belt.

Additionally or alternatively, it is preferred that the radiation source is tunable with respect to the wavelength of the emitted radiation, in particular by means of a controller. This may enable, for example, the selection of a wavelength that is advantageous for disinfection.

Additionally or alternatively, it is preferred that the irradiation device source comprises a semiconductor radiation source, wherein preferably the irradiation device comprises at least one LED (i.e. one LED or a plurality of LEDs).

Such UVC LED or other semiconductor UVC radiation source may, for example, be considered to be an efficient radiation source and/or a radiation source that may be operated in relatively close proximity to said item requiring disinfection and hence operated with high intensity. This is because conventional UVC sources such as mercury lamps do not only contain toxic mercury but are also costly to operate due to their high energy consumption. Moreover, they are usually rather bulky which makes it difficult to arrange them close to the items. According to the inverse-square law, an inverse square relationship exists between the distance from the radiation source and the irradiance value. Due to the distance from the objects and the resulting lower irradiation efficiency, the mercury lamps which already have a poor energy yield must emit a particularly high radiation dose which makes their use even more inefficient. In addition, such lamps are often unreliable and cannot be run over many hours without stoppage thereby causing stoppages of the production process.

Additionally or alternatively, the UVC radiation preferably has a wavelength of at least 150 nm and/or a wavelength of at most 280 nm, more preferably a wavelength of at least 200 nm and/or a wavelength of at most 280 nm, most preferably a wavelength of at least 260 nm and/or a wavelength of at most 280 nm.

Additionally or alternatively, the disinfection device according to the invention preferably further comprises a disinfectant application device for applying a disinfectant to the items to be subjected to disinfection, wherein the disinfection device is adapted to apply the disinfectant to the items to be subjected to disinfection prior to and/or during and/or after the irradiation with the UVC radiation. Using both UVC radiation and a disinfectant may for instance have a synergistic effect such as a disinfection efficiency that is considerably high compared to the use of only UVC radiation or only a disinfectant. For instance, it may be possible to achieve an efficient disinfection by applying a concentration of the disinfectant that is much lower than a concentration that would be conventionally used in a method without additional irradiation.

More preferably, the disinfectant is at least one of

- hydrogen peroxide, in particular aerosolised hydrogen peroxide,
- peracetic acid,
- alcohol or an alcohol water mixture, in particular isopropanol and/or ethanol or a mixture of water with isopropanol and/or ethanol.

Herein, for instance hydrogen peroxide may be used in a concentration between 0.25% and 8%, in particular between 0.5% and 3%. In this context, a concentration of 0.25% means that the items to be disinfected are treated with a medium (e.g. gas atmosphere, aerosol, etc.) comprising 0.25 weight percent of hydrogen peroxide.

The optional disinfectant application device may comprise a chamber for accommodating the items during the application of the disinfectant. If the disinfection device comprises a tunnel (see below), it is preferred that the chamber is connected to the tunnel, more preferably through an orifice.

Additionally or alternatively, the disinfection device according to the invention preferably further comprises a gas flow device for applying a gas flow to the items to be subjected to disinfection treatment, wherein more preferably the gas flow device is a gas blade device, and/or wherein more preferably the gas flow is an air flow, in particular a flow of purified air, especially a flow of disinfected air. By applying a gas flow to the items, for example dust may be removed from the items' surface. Dust particles are a barrier to radiation, which could result in insufficient disinfection in a surface area below a dust particle. The gas flow device may in addition be used, for example, as an access barrier or access control to prevent items with oversized height and/or width from entering the portion of the disinfection device in which the UVC radiation sources are arranged such as the optional tunnel described below. This access barrier or access control may be of particular benefit when using the disinfection device with vertical and/or horizontal adjustable UVC radiation sources. It is possible that also the gas flow device is adjustable with respect to its position and in particular that the arrangement of the gas flow device is adjustable with respect to the shape and size of the items to be disinfected. The optional adjustability of the UVC radiation sources will be further discussed below.

Additionally or alternatively, the disinfection device according to the invention preferably is adjustable with respect to the horizontal distance between the radiation source or at least a part of multiple radiation sources and the items to be subjected to disinfection treatment. Herein, the horizontal distance is the distance in a direction perpendicular to the direction of the conveyor direction and the vertical direction. In other words, the horizontal distance is the lateral spacing between the radiation source(s) and the items. More preferably, the irradiation device is in addition adjustable with respect to the vertical distance between the radiation source or at least a part of multiple radiation sources and the items to be subjected to disinfection treatment.

Herein, a small distance between a radiation source and an item to be irradiated may be for example enhance the irradiation efficiency in view of the inverse square relationship mentioned above.

For this horizontal and/or vertical adjustment, the disinfection device may comprise a movement device for moving the radiation source or at least a part of multiple radiation sources and thereby adjust the horizontal distance and/or the vertical distance, wherein the movement device may for example comprise an actuator controlled by an electrical selector switch and/or a control unit. Examples for actuators that may be used for this task are: electric stepper motor, electric linear actuator, pneumatic actuator, hydraulic actuator.

Additionally or alternatively, the disinfection device according to the invention preferably comprises at least one cassette, wherein the cassette or each of a plurality of cassettes accommodates the radiation source for emitting the UVC radiation. For instance, such a cassette may accommodate a plurality of UVC emitting LED.

Herein, preferably at least a wall portion of the cassette has high transparency to the UVC radiation so that the cassette may have a closed design but nevertheless allows radiation to be emitted from the cassette.

If such a cassette is used, the disinfection device may further comprises a cassette gas flow device for generating a gas flow (e.g. air such as purified air, in particular disinfected air) flowing through the cassette. Such a gas flow can have, for example, a cooling effect to cool the LEDs.

Such cassettes are preferably provided with reflective surfaces for directing the radiation towards the items to be disinfected.

Such cassettes not only provide protection for the radiation source, but may, for example, also enable simplified handling, in particular simplified replacement of the radiation source, and, depending on the design, effective radiation guidance and/or effective cooling.

Additionally or alternatively, the disinfection device according to the invention preferably comprises at least one optical magnification device placed between the radiation source or one of multiple radiation sources and the items to be subjected to disinfection treatment, wherein, more preferably, the magnification device comprises a lens, in particular a convex lens, especially a spherical lens, made of a material that is transparent to the UVC radiation, in particular quartz glass.

Additionally or alternatively, preferably, the conveyor speed is adjustable, in particular by an electrical selector switch and/or the control unit. By adjusting the conveyor speed, for example the radiation dose may be adjusted because due to the comparatively long irradiation time, items that are moved slowly alongside a radiation source receive a higher overall radiation dose compared to a fast movement.

Additionally or alternatively, the disinfection device according to the invention preferably further comprises a tunnel with an entry orifice and an exit orifice, wherein the conveyor is adapted to convey the items to be subjected to disinfection treatment from the entry orifice to the exit orifice, and wherein the UVC radiation is emitted in the interior of the tunnel. More preferably the tunnel comprises internal surfaces that are reflective to the UVC radiation.

Such a tunnel may, for example, provide an at least mostly enclosed area for the irradiation and allow the items to be transported from one location (tunnel entrance) to another location (tunnel exit).

If the disinfection device according to the invention comprises both a tunnel and a gas flow device, preferably the latter is configured to generate a gas flow flowing in the direction from the exit orifice to the entry orifice. This gas flow direction can be particularly advantageous, for example, if the disinfection device is used to introduce items into a cleanroom or the like, as the inflow of possibly contaminated gas into the cleanroom is suppressed.

Preferably, the disinfection device according to the invention is configured such that there is no unacceptable exposure of personnel to UVC light and that in particular the recommended exposure limits are complied with. For this task, the disinfection device according to the invention may further comprise an entry orifice curtain and/or an exit orifice curtain, wherein entry orifice curtain is at least partially non-transparent to the UVC light and/or wherein exit orifice curtain is at least partially non-transparent to the UVC light. The entry orifice is coverable with the entry orifice curtain such that the UVC light is at least partially prevented from leaving the tunnel via the entry orifice. The exit orifice is coverable with the exit orifice curtain such that the UVC light is at least partially prevented from leaving the tunnel via the exit orifice. Because the curtains are moveable, items may nonetheless pass through the entry orifice and the exit orifice, respectively.

Additionally or alternatively, the disinfection device according to the invention preferably comprises at least four radiation sources whereof

- at least two are arranged at either side of the conveyor with respect to the direction of conveyor movement to horizontally irradiate the items to be subjected to disinfection treatment,
- at least one is arranged above the conveyor to vertically irradiate the items to be subjected to disinfection treatment from above, and
- at least one is arranged underneath the conveyor to vertically irradiate the items to be subjected to disinfection treatment from below, the conveyor being at least partially transparent to the UVC radiation.

Such an arrangement of the radiation sources allows, for example, effective irradiation of the entire surface of an item to be disinfected.

Additionally or alternatively, the disinfection device according to the invention preferably comprises an image and/or size recognition device including a camera and/or a sensor, wherein the disinfection device is configured to be adjusted according to an image or multiple images of and/or a size information about the items to be subjected to disinfection. More preferably, adjusting the disinfection device includes adjusting at least one of the following properties:

- horizontal distance between the radiation source or at least a part of multiple radiation sources and the items to be subjected to disinfection treatment,
- vertical distance between the radiation source or at least a part of multiple radiation sources and the items to be subjected to disinfection treatment,
- conveyor speed,
- UVC emission power of the radiation source or at least a part of multiple radiation sources.

By means of such an adjustment, the disinfection device may, for example, be adjusted precisely and/or automatically in such a way that the disinfection is particularly effective.

Additionally or alternatively, the disinfection device according to the invention preferably comprises a humidity control device adapted to maintain the relative humidity in at least a portion of the area in which the items are irradiated below a predefined threshold, wherein the threshold is 65 %, preferably 50 %, more preferably 40 %.

Preferably, the items to be subjected to disinfection are irradiated with a UVC light dose of at least 50 mJ/cm$^2$, more preferably at least 100 mJ/cm$^2$, most preferably at least 200 mJ/cm$^2$, in particular at least 500 mJ/cm$^2$. By using such doses, for instance a sufficient Log reduction may be achieved and/or cellular repair processes within the microorganisms may be sufficiently prevented. According to specific embodiments of the present invention, a chemical disinfectant, such as hydrogen peroxide, is used in addition to the UVC radiation. Herein, low concentrations of the disinfectant, e.g. 1% of hydrogen peroxide, are preferred. Low concentrations may be sufficient because of the UVC radiation.

The system according to the invention is a system comprising

- a cleanroom and

- a disinfection device according to the invention,

wherein the disinfection device is configured and/or positioned to subject items to be introduced in the cleanroom to a disinfection treatment.

The system according to the invention thus provides a cleanroom system with a device for introducing items required in the cleanroom, whereby the items are disinfected during transportation. The above-described features and advantages of the disinfection device according to the invention are therefore advantageously used to load a cleanroom with disinfected items.

The use according to the invention is a use of the disinfection device according to the invention as a transport connection between a first room and a second room,

wherein items are transportable from the first room into the second room through the disinfection device, so that the items are irradiated with the UVC radiation during their transport. Preferably, the first room and the second room are separated from each other apart from the connection through the disinfection device.

By this use, it may be achieved, for example, that items transported from the first room to the second room such that they arrive in the latter in a disinfected state. In this way, for example, lower bioburden conditions may be maintained in the second room.

The method of disinfecting items according to the invention is a method comprising the steps of

- transporting the items in a forward direction using a conveyor, preferably transporting the items within a tunnel from an entry orifice to an exit orifice,
- irradiating the items with UVC radiation while being transported, and
- guiding the items to be subjected to disinfection treatment while being conveyed in a direction that is other than the conveyor direction.

The Method of introducing items into a cleanroom according to the invention is a method comprising the steps of

- transporting the items in a forward direction from outside the cleanroom into the cleanroom using a conveyor, preferably transporting the items within a tunnel from an entry orifice communicating with the outside of the cleanroom to an exit orifice communicating with the interior of the cleanroom,
- irradiating the items with UVC radiation while being transported, and
- guiding the items to be subjected to disinfection treatment while being conveyed in a direction that is other than the conveyor direction.

These methods may, for example, be carried out by means of the disinfection device according to the invention or the system according to the invention. These methods may have the advantages described above for the disinfection device according to the invention.

Other features and expediencies of the invention may be found in the description of exemplary embodiments with the aid of the appended drawings.

Fig. 1    is a schematic isometric view of the disinfection device according to a first embodiment of the invention.

Fig. 2    shows three schematic sections through the disinfection device according to the first embodiment. Fig. 2 (a) is a horizontal section. Fig. 2(b) is a vertical section parallel to the conveyor direction. Fig. 2 (c) is a vertical section perpendicular to the conveyor direction.

Fig. 3    shows two schematic sections through the disinfection device according to the first embodiment. Fig. 3 (a) shows a region of a vertical section parallel to the conveyor direction showing further details of the disinfection device. Fig. 3 (b) shows a vertical section perpendicular to the conveyor direction showing further details of the disinfection device.

Fig. 4    is a schematic section through the disinfection device according to a second embodiment of the invention, wherein the section is a vertical section perpendicular to the conveyor direction.

Fig. 5    shows a region of a schematic section through the disinfection device according to a third embodiment of the invention, wherein the section is a vertical section parallel to the conveyor direction.

Fig. 6    shows two schematic sections through the disinfection device according to the invention showing details of further embodiments of the invention. Fig. 6(a) is a horizontal section. Fig. 6(b) is a vertical section parallel to the conveyor direction.

Fig. 7    shows a region of a schematic section through the disinfection device according to the invention showing details of further embodiments of the invention, wherein the section is a vertical section parallel to the conveyor direction.

Fig. 8    shows a plan view of a region of a conveyor belt included in the disinfection device of further embodiments of the invention.

Fig. 9    is a schematic section through the disinfection device according to the invention showing details of further embodiments of the invention, wherein the section is a vertical section perpendicular to the conveyor direction.

Fig. 10   is a schematic sectional view of a system according to a further embodiment of the invention.

Exemplary embodiments of the invention will be described below with reference to the figures. Herein, the reference numbers used in the figures are included in the description in parentheses.

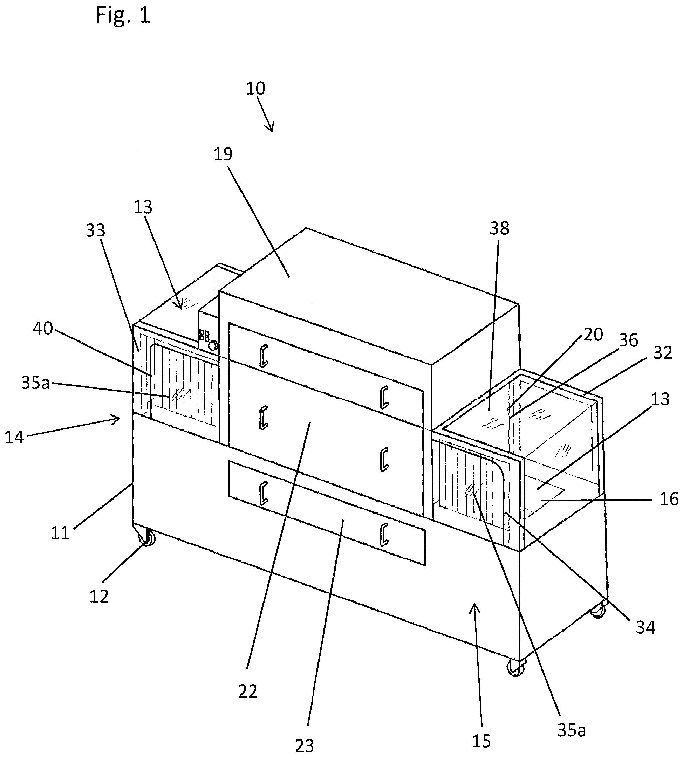

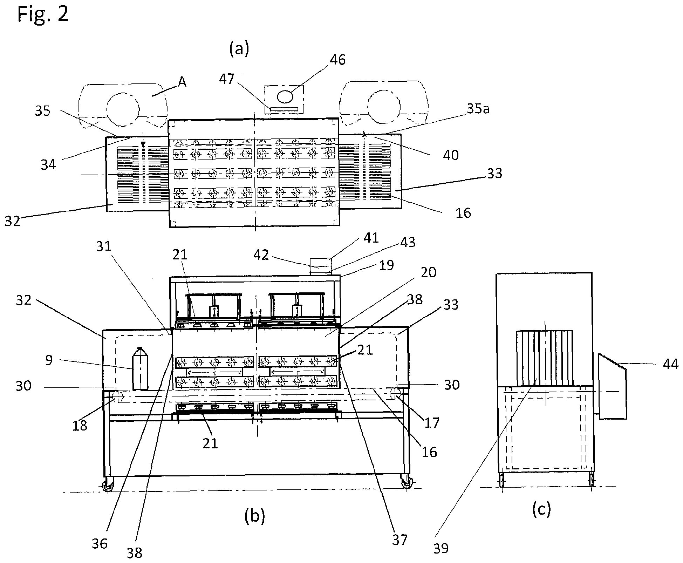

Fig. 1 is a schematic isometric view of the disinfection device (10) according to a first embodiment of the invention. Fig. 2 shows features of this disinfection device in three schematic sections.
Given the nature of the intended application, the external and work surfaces of the disinfection device (10) are compatible with standard cleaning and disinfection agents including ethanol, isopropanol or other alcohols, alcohol-based solutions, hydrogen peroxide solutions, detergents, etc.
The device (10) comprises a lower support (11) such as a frame or casing. The lower support is preferably, although not necessarily, mounted on lockable castor wheels (12) or the like. Castor wheels or similar devices allows the disinfection device to be portable.
Mounted on the lower support (11) is a conveyor assembly (13) with a conveyor that travels substantially the length of the device (10) with the direction of the conveyor defining the entry site (14) of the device where items to be subjected to disinfection are inserted and the exit site (15) of the device where the items leave the device after disinfection. In Fig. 2 (a), by way of example, personnel (A) inserting items into the disinfection device (10) and taking items out of the disinfection device (10) is shown. Alternatively, the insertion and/or the discharge of items can also be achieved automatically without manual intervention by personnel, for example by transferring items from/to transport devices coupled to the disinfection device (10). The conveyor assembly (13) comprises a conveyor including a conveyor belt (16) which is connected to two or more rollers (17 and 18) positioned either end of the device (10) and driven by a suitable means such as a motor and preferably a gearbox (not shown). In operation, the belt (16) rotates in a continuous loop operation around the rollers (17 and 18).
The device (10) comprises an irradiation device for irradiating the items (9) with UVC radiation while the items (9) are transported by the conveyor. In the first embodiment, the irradiation device comprises at least one UVC light.
The device (10) comprises a tunnel (20). In the context of the present invention, a tunnel is understood as a housing or other hollow structure that has at least two openings (orifices) and is at least essentially closed otherwise. The tunnel (20) may be formed by a top frame (19) mounted around the conveyor assembly (13). Inside the tunnel (20), at least on UVC lights (21), i.e. a radiation source emitting UVC radiation, is arranged.

Preferably, at least four UVC lights (21) are positioned inside the tunnel (20), one at each longitudinal side of the conveyor assembly (13), one at the roof of the tunnel (20) and one underneath the conveyor assembly (13), thereby shining UVC light inside and on four sides of the tunnel (20) around the conveyor to ensure an irradiation of the items to be subjected to disinfection. In this case, the irradiation device comprises at least four UVC lights.

More preferably, arrays of multiple UVC lights (21) are used as shown in Fig. 2. In this case, the irradiation device comprises arrays of multiple UVC lights.

Preferably, the inside surfaces of the tunnel (20) are made from polished stainless steel or another surface with relatively high light reflective properties to maximise the UVC intensity inside the tunnel (20). Herein, polished stainless steel may for instance be a good compromise between UVC reflectivity and corrosion resistance.

Preferable materials of construction of those elements of the disinfection device that become irradiated with the UVC radiation such as the inside walls of the tunnel (20) itself and the elements within the tunnel (20) are UVC proof such as PTFE, stainless steel, or suitable fluoropolymers to avoid material degradation from extended periods of UVC light exposure.

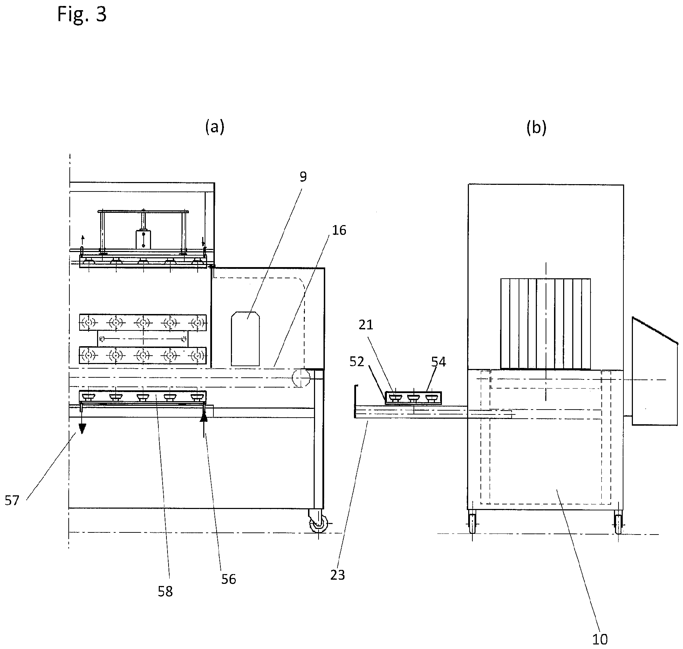

Preferably, the UVC lights (21) are mounted in outwardly extending drawers (22, 23) for ease of maintenance and replacement. In Fig. 3 (b), one of these drawers (23) is shown in an open state, in which the UVC lights (21) can be easily accessed for maintenance and replacement. The lights (21) shown in Fig. 3 (b) are accommodated in a cassette (52) covered by a window (54) transparent to the UVC radiation. The cassette (52) will be further discussed below.

Preferably, the tunnel (20) has a length of around 1 m to 2 m with UVC lighting from the top, the bottom, and sides such that the entire inner tunnel (20) or a majority of its length is directly illuminated by UVC light. Shorter and longer tunnel lengths are also anticipated, dependant on the time of exposure required by the application and the intended conveyor speed.

The conveyor belt (16) preferably comprises a mesh of stainless steel or non-metallic wire or grid to provide a high degree of light exposure to the item (9) also through the conveyor belt (16).

In operation, items (9) for disinfection are placed on the conveyor belt (16) and travel through the tunnel (20). The speed of the conveyor belt (16) is adjusted to ensure the microorganism is exposed to a sufficient dose of UVC light to achieve the desired Log reduction.

Preferably, the disinfection device (10) comprises electronic e-stops (30) (emergency stops) at each end of the conveyor belt (16) which for instance automatically stop the conveyor belt (16) if the items (9) hit said e-stop (30) and build up without being taken off the conveyor belt (16).

Preferably, the disinfection device (10) comprises at least one gas flow device such as an air blade (31) mounted at the entry point of the tunnel (20) to direct an air flow or a flow of another gas, in particular filtered air or other gas, towards the conveyor belt (16) and items (9) positioned on the conveyor belt (16). The air blade (13) or other gas flow device may be used to remove contaminants and dust particles from the items (9) before they enter into the tunnel (20). This blowing off of particles is not only a cleaning step, which per se may contribute to the disinfection effect because, for example, microbes adhering to the items are removed. In addition, a particle that is present at a position on the surface of the item causes a shadow effect as it prevents the UVC radiation from reaching this position on the surface or weakens the radiation. By removing particles, this shadow effect is reduced or even completely avoided, thus increasing the effectiveness of the irradiation.

Additionally or alternatively, the air blade (31) or other gas flow device may be adjustable in the vertical and/or horizontal direction. This makes the blowing-off effect more efficient. This also allows it to be employed as an access barrier or access control to prevent items with oversized height and/or width from entering the tunnel (20). This access barrier or access control may be of particular benefit when using the disinfection device (10) with vertical and/or horizontal adjustable lights (21). The possible adjustability of the lights (21) will be further discussed below.

As can be seen in Figures 1, 2 (a) and 2 (b), the disinfection device (10) comprises an entry guard (32) and an exit guard (33). Said guards (32, 33) are preferably transparent housings with one or more access slots (34, 40) which is substantially perpendicular to the direction of the conveyor movement ("conveyor direction"). Preferably, said guards (32, 33) are made from a non-UVC transmitting material such as Perspex which acts to provide excellent operator visibility as well as excellent UVC shielding. It is noted that the entry guard (32) is shown on the right side of the device (10) and the exit guard (33) on the left side in Fig. 1, whereas the entry guard (32) is shown on the left side of the device (10) and the exit guard (33) on the right side in Fig. 2 (a) and Fig. 2(b). This is only a question of the positioning the disinfection device (10) and is irrelevant for its structure and functioning. The perpendicular orientation of said access slots (34, 40) provides a good level of operator shielding from direct UVC light beams which may be released from the tunnel (20) as the disinfection item travels through the moveable tunnel curtains (38, 39). The guards (32 and 33) are typically manufactured from a transparent plastic which blocks UVC transmission therefore helping to shield the operators from stray UVC light.

The conveyor is configured to take items placed in the entry guard (32) and convey them through the tunnel (20) into the exit guard (33). That is, the conveyor reaches into the guards (32, 33).

The entry guard (32) is connected with to tunnel (20) via an entry orifice (36) through which the items to be disinfected enter tunnel (20) when being transported through the disinfection device (10) by the conveyor. The exit guard (33) is connected to the tunnel (20) via an exit orifice (37) through which the items to be disinfected leave the tunnel (20) and enter the exit guard (33) when being transported through the disinfection device (10) by the conveyor.

The entry orifice (36) and exit orifice (37) and thereby the interfaces between the guards (32, 33) and the tunnel (2) are covered by moveable tunnel curtains (38) and (39). By way of example only, said tunnel curtains (38 and 39) are strips of UVC opaque material designed such that they allow the disinfection item (9) to physically enter the tunnel (20) but help prevent UVC light (21) from escaping the tunnel (20). Alternatively or additionally, other forms of curtains and/or shutter doors, in particular automatic shutter doors, may be installed to cover the entry orifice (36) and/or the exit orifice (37). Alternatively, it is also possible that no curtain or door is installed at the entry orifice (36) and/or exit orifice (37), in particular if the personnel is sufficiently protected from the UVC radiation by other means such as perpendicularly arranged access slots (34, 40) and/or moveable curtains (35, 35a) covering the access slots (34, 40).

A set of moveable curtains (35, 35a) of similar material and construction as described for the aforementioned curtains (38, 39) may be provided, wherein these curtains (35, 35a) cover the entry guard (32) access slot (34) and exit guard (33) access slot (40).

Preferably, the disinfection device (10) comprises a fan system (42), which is an example of a gas flow device that pushes air from the external area of the device (10) to the internal area of the tunnel (20). More preferably, a coarse pre-filter (41) positioned on top of the top frame (19) prevents large contaminants being drawn into the fan (42) system, whereas an appropriate filter (43) such as a HEPA filter communicates between the inner area of the tunnel (20) and the fan (42).

By using such fan system (42), the disinfection device (10) has a filtered air flow through the tunnel (20). The direction of air travel is preferably from the tunnel exit end (37) to the tunnel entry end (36), in a contraflow direction to the conveyor belt (16) movement. Such contraflow air movement helps to ensure that contaminants do not pass from the entry (36) side, which is generally classed as a less clean side, to exit (37) side of the tunnel (20), which is generally classed as a cleaner side.

Preferably, an HMI (Human Machine Interface) control panel (44) is mounted on the device (10). Such control panel is shown, by way of example only, in Fig. 2 (c). Various variables and parameters may be indicated by a display of the control panel, e.g. conveyer speed, UVC light source power, UVC light source failure indicator, HEPA filter differential pressure indicator, power on/off, fan on/off, UVC lights on/off, etc., for instance for providing full secure operational control for the intended application. A full integral emergency stop safety system is possible, as indicated by way of example only, by the stop button (46) shown in Fig. 2 (a).

Preferably, the device (10) also contains an audible alarm (47) indicating filtered air flow high or low, high filter pressure differential, and/or other failure status of the device (10).

As shown in Figures 1 to 3, the disinfection device (10) according to the first embodiment - or, more precisely, the irradiation device of the disinfection device - comprises multiple individual UVC lights (21). One or more of these individual UVC light elements (21) are mounted in a cassette (52) which facilitates light replacement/maintenance and protects the UVC light elements (21). The disinfection device (10) comprises multiple cassettes (52). According to other embodiments, the disinfection device (10) may also comprise only one cassette (52) and/or UVC light elements (21) that are not mounted in cassettes (52).

Such light cassette (52) comprises a case (53), again with highly reflective surface properties, and a substantially transparent window (54) which protects the light elements (21). This window (54) is preferably manufactured from a material to maximise UVC transmission across such as quartz glass or FEP (fluorinated ethylene propylene).

The UVC transparent protective window (54) may be adapted to contain a light element breakage within the cassette whilst allowing a high transmission of UVC, in particular a transmission of >75%. High transmission may improve the speed and efficacy of the UVC light disinfection. Additionally or alternatively, for instance UVC light elements (21) with a protective shatter proof UVC transparent coating and/or individually covered UVC light elements (21) are also possible in the context of the present invention.

The use of highly polished surfaces, and additional reflectors (not shown) increases the UVC light (21) directed to the surface of the item (9) to be disinfected on the conveyor belt (16). When incorporated, the reflectors are angled such that light is directed downwards to the surface of the conveyor belt (16) providing broad light coverage.

Preferably, filtered air or other gas enters the light cassette (52) from a cassette gas entry point (56) and travels across the light elements (21) to exit the light cassette (52) at a cassette gas exit point (57). This air flow helps to remove heat generated from the light elements (21) which may prolong operational life and increases UVC light intensity. The output and stability of UVC intensity may fall as temperature increases. In particular temperatures maintained at or below 30°C may provide a stable system but as temperatures approach 40 °C the light intensity may begin to drop substantially.

Preferably, a spherically dimpled surface (58) may be provided at the inner surfaces of the cassettes (52), which are in particular reflective surfaces. Such spherically dimpled surface (58) may help to create a turbulent air flow thereby maximising the heat exchange process between the air and the light elements (21).

It is preferred to use UVC radiation sources with highly reflective backsides and/or additional reflectors guiding the radiation towards the item to be irradiated.

The disinfection device (10) comprises a guiding device for guiding the items to be subjected to disinfection treatment while being conveyed in a direction that is other than the conveyor direction. The guiding device is not shown in Figures 1 to 3.

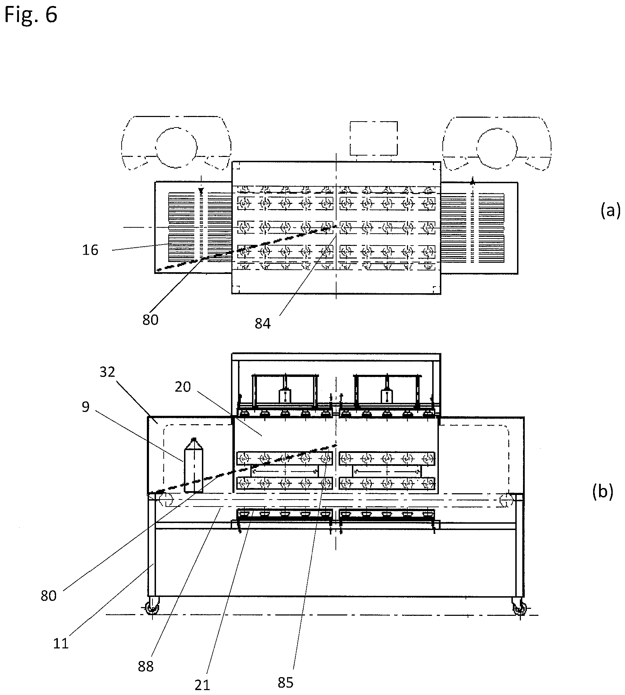

The guiding device of the disinfection device (10) according to the first embodiment is shown in Fig. 6 (a) and Fig. 6 (b), which correspond to Fig. 2 (a) and Fig. 2 (b) and show a nudge wire (80) as an example of the guiding device being part of the disinfection device.

One end of the nudge wire (80) may be mounted on the static lower support (11) of the device (10). Preferably, the nudge wire (80) is mounted in an inclined direction. Preferably, the nudge wire (80) starts in the entry guard (32) as shown in Fig. 6 or at the entry orifice (36) of the tunnel (20) and proceeds in a longitudinal and lateral manner towards the centre (84) of the tunnel (20) as shown in Fig. 6 or beyond, raising vertically in height (85) along the length of the device (10).

As items (9) for disinfection travel along the conveyor belt (16), which preferably is a meshed conveyor belt, the nudge wire (80) or other guiding device moves them across the conveyor mesh belt (16). That is, the items (9) are moved in the conveyor direction by means of the conveyor and moved, in particular moved in a sliding manner, in a direction across the conveyor direction by means of the guiding device. This movement of an item (9) in addition to the conveyor movement exposes the previously seated and covered surfaces to the UVC lights (21), therefore eliminating the shadowing effect of the conveyor mesh belt (16) on said item (9).

Preferably, support braces (88) are provided that are laterally and/or longitudinally angled underneath the conveyor belt (16). As said braces (88) are not straight and in line with the longitudinal axis of the conveyor belt (16), this again has the effect to reduce the light shadowing on the item (9).

More preferably, the support braces (88) are hygienically profiled and provided with substantially curved surfaces adjacent to the conveyor belt (16) so that dust, debris and contaminants do not naturally sit on the surface of said braces (88) causing hygiene issues.

It is possible that more than one nudge wire (80) is provided.

Additionally or alternatively, different guiding devices fulfilling the function of the nudge wire (80) described above, may be provided, such as one or more nudge bars, one or more nudge sheets, one or more guiding plates, one or more wedge structures, etc.

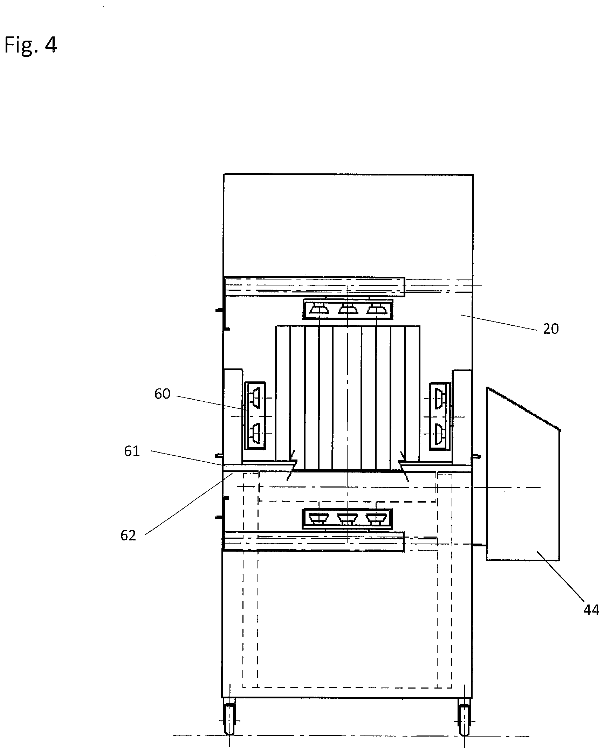

Fig. 4 shows a sectional view of the disinfection device (10) according to a second embodiment of the invention, wherein the section is a section through the tunnel (20). Apart from the peculiarities described below, the disinfection device (10) according to the second embodiment corresponds to the disinfection device (10) according to the first embodiment so that for the description of these features and optional features, reference is made to the description above. The guiding device being part of the disinfection device is not shown in Fig. 4. For a description of the guiding device, reference is made to the description of the first embodiment and Fig. 6 showing an angled nudge wire (80) as an example of the guiding device.

According to the second embodiment, the device (10) comprises UVC light cassettes (60) that have fixed operating positions.

Preferably, one or more of the fixed UVC light cassettes (60) are mounted in one or more outwardly moveable drawers (61).

More preferably, such a drawer (61) is in communication with a proximity switch (62), which is activated by opening of the drawer (61) and which then communicates with the control panel (63) to turn off the UVC lights and/or the conveyor power. This functionality may increase operator safety. Such an outwardly moveable drawer (61) may further facilitate the maintenance of the device (10) such as UVC light cassette (60) replacement.

According to the second embodiment, the cassettes (60) are arranged underneath the conveyor belt (16), which is at least semi-transparent to the UVC radiation, above the conveyor belt (16), and at either side of the conveyor belt (16) to ensure an irradiation of the items (9) to be disinfected from all sides. At each of these positions (underneath, above, and at the sides of the conveyor belt), one or more than one cassette (60) may be arranged. Each cassette (60) may have a longitudinal extension that essentially corresponds to the length of the tunnel (20) or a shorter length.

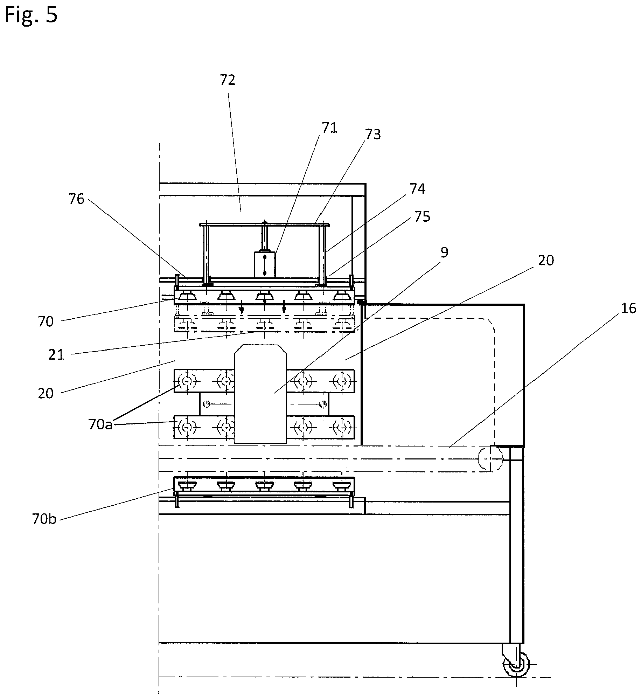

Fig. 5 shows a sectional view of the disinfection device (10) according to a third embodiment of the invention, wherein the section is a section through the tunnel (20) of. Apart from the peculiarities described below, the disinfection device (10) according to the second embodiment corresponds to the disinfection device (10) according to the first and second embodiments so that for the description of these features and optional features, reference is made to the description above. The guiding device being part of the disinfection device is not shown in Fig. 4. For a description of the guiding device, reference is made to the description of the first embodiment and Fig. 6 showing an angled nudge wire (80) as an example of the guiding device.

According to the third embodiment, the device (10) comprises UVC light cassettes (70, 70a) that have adjustable operating positions.

Similar to the second embodiment, one or more of the UVC light cassettes (70, 70a, 70b) may be mounted in at least one outwardly moveable drawer to facilitate the maintenance of the device (10).

According to the third embodiment, the cassette (70b) is arranged underneath the conveyor belt (16), which is at least semi-transparent to the UVC radiation, the cassette (70) is arranged above the conveyor belt (16), and the cassettes (70a) are arranged at either side of the conveyor belt (16) to ensure an irradiation of the items (9) to be disinfected from all sides. At least the positions of the cassettes (70, 70a) arranged above the conveyor belt (16) and at either side of the conveyor belt (16) are adjustable. At each of these positions (underneath, above, and at the sides of the conveyor belt), one or more than one cassette (70, 70a, 70b) may be arranged. Each cassette (70, 70a, 70b) may have a longitudinal extension that essentially corresponds to the length of the tunnel (20) or a shorter length.

In the third embodiment of the invention, the positions of the UVC light cassettes (70) and (70a) in the tunnel (20) are adjustable so that they can maintain close proximity to the item (9) to be disinfected. The adjustment may for instance be achieved by using automated mechanical, electrical, hydraulic or pneumatic linear actuator means. Herein, it is preferred that the positions of the cassettes (70, 70a) can be adjusted such that their distance from the items (9) to be disinfected is in the range of 10 to 20 cm or below.

Reducing the distance between the item (9) and the radiation source (21) may result in an increase of the efficiency of the irradiation. The inventor has found that for some organisms it may be advantageous that, when delivering the required fluence of UVC light, to have a higher irradiance and lower time of exposure.

Preferably, the adjustment movement of the cassette (70) arranged above the conveyor belt (16) is achieved by a movement device having the following configuration: A pneumatic piston (71) is connected to a moving platform assembly (72). Said moving platform assembly comprises of a platform (73) connected to two guide rods (74) which slide through bushes (75) mounted in the fixed platform (76). The guide rods (74) are connected to the light cassette assembly (70), therefore as the pneumatic piston (71) moves vertically, it has a direct and equal vertical movement on the light cassette assembly (70). Alternatively, different kinds of actuators may be used to achieve the adjustment movement of the cassette (70) arranged above the conveyor belt (16).

Preferably, the side cassettes (70a) are inwardly movable towards the conveyor belt (16) by mechanical means (not shown in Fig. 5). For instance, said mechanical means may comprise a spring-plunger mounted on a moving plinth containing the cassette or cassettes (70a). The spring-plunger locates in predetermined orifices thereby allowing the operator to quickly and repeatedly position the cassette or cassettes (70a) on the disinfection device (10).

The adjustment of the positions of the cassettes (70, 70a) can be carried out by the personnel, for example, via respective settings on the control panel or by manual intervention. It is also possible for these adjustments to be made automatically, for example in response to a determination of the dimensions of the items to be disinfected (9) or an automatic image recognition. The adjustment of the positions of the cassettes (70, 70a) can also be carried out partially by personnel and automatically.

At least one cassette (70b) arranged underneath the conveyor belt (16) is preferably fixed in its vertical position, more preferably in close proximity to the conveyor belt (16).

According to further embodiments of the invention not illustrated in the figures, only the position of the cassette(s) arranged above the conveyor belt (16) is adjustable.

According to further embodiments of the invention not illustrated in the Figures, also the position of the at least one cassette arranged underneath the conveyor belt (16) is adjustable.

To compensate for a possible shadowing effect resulting from the conveyor belt (16), higher intensity UVC lights (70) can be used. Additionally or alternatively, the disinfection device (10) may incorporate anti-shadowing technology, for instance an anti-shadowing technology described below.

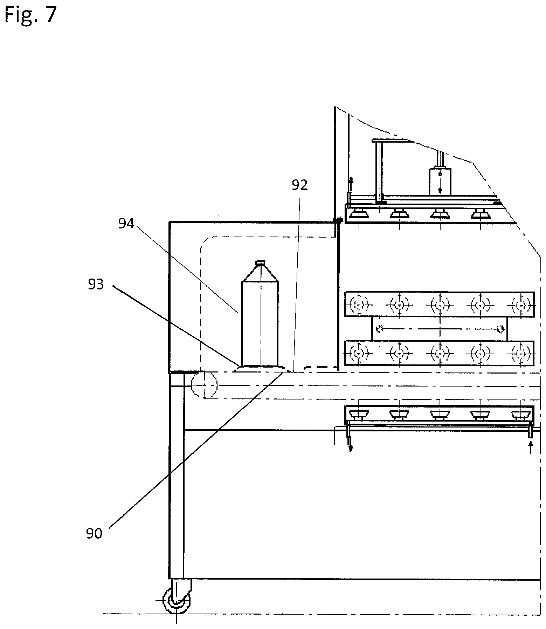

Fig. 7 is based on Fig. 2 (b) and demonstrates the additional features according to further embodiments of the invention on the basis of the first embodiment by way of example only. These additional features may be implemented in any embodiment of the invention.

The disinfection device (10) according to these further embodiments comprise a meshed conveyor belt (90) having flat sections (92) and raised sections (93) across its length and width. The raised sections (93) act to suspend items (94) off the basis of meshed conveyor belt (90) surface and again reduce UVC light shadowing. The protrusions constituting the raised sections are preferably made of a material that has at least a certain transparency to UVC radiation. The disinfection device (10) according to these further embodiments may be particularly advantageous as tests have shown that UVC light shadowing can be considerably reduced by using such conveyor belt design.

The conveyor belt design according to these embodiments is combined with the provision of a nudge wire (80) or another guiding device as described above in order to further reduce the UVC light shadowing.

According to further embodiments of the invention that are not illustrated in the figures, the disinfection device (10) comprises an at least substantially solid conveyor belt made of a material that is sufficiently UVC transmitting. The solid UVC transmitting belt allows UVC light to pass through the surface of the belt, again, being advantageous to provide high microorganism Log reduction on the seated surface of the item.

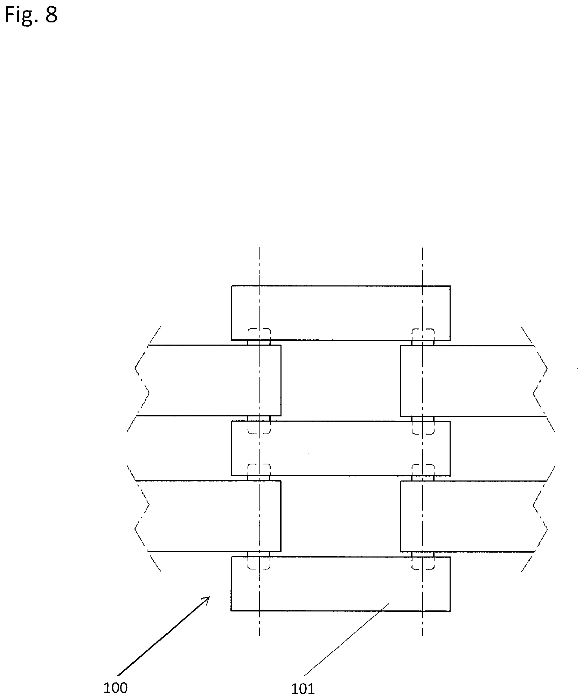

Fig. 8 illustrates the design of the conveyor belt (100) according to further embodiments of the invention. This conveyor belt (100) comprises a plurality of links (101) connected to each other, preferably forming a mesh. Preferably, the links (101) are made from a material with UVC light transmitting properties. Such conveyor belt design, which may be used in any of the disinfection devices according to the embodiments of the invention, is a hybrid design in that it may have a reduced shadowing effect or no shadowing at all at all and may at the same time provide the advantages of a mesh belt to carry heavier items for disinfection.

The disinfection device according to specific embodiments of the invention comprises at least one LED UVC light element, preferably a plurality of LED UVC light elements. LED UVC light elements may be used in any of the disinfection devices according to the embodiments described above. LED UVC light elements are not only environmentally friendly as their energy efficiency is relatively high and as they avoid the use of mercury. In addition, the LEDs generate far less heat, provide an instant light source and are less fragile and therefore less prone to light element breakage as compared to, e.g., mercury lamps. LED UVC lights may also have their wavelength tuned to an optimum range for microorganism deactivation.

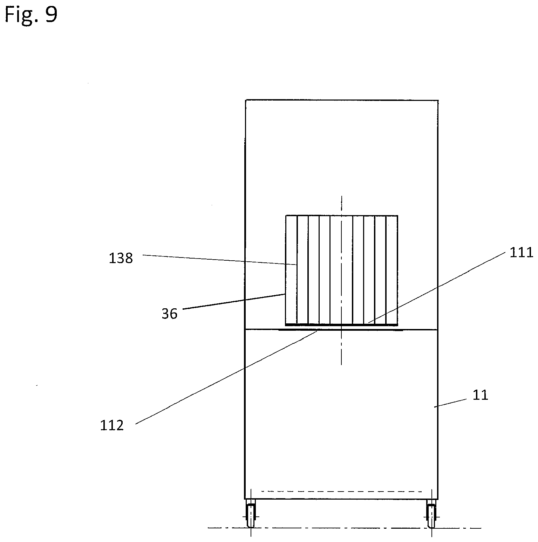

Fig. 9 shows the split light protection curtain (138) covering the entry orifice (36) according to further embodiments of the invention. Such curtain (138) may be implemented in any of the embodiments of the invention as

- a curtain covering the entry orifice (36) of the tunnel (20) and/or
- a curtain covering the exit orifice (37) of the tunnel (20) and/or
- a curtain covering the access slot (34) of the entry guard (32) and/or
- a curtain covering the access slot (40) of the exit guard (33).

The curtain (138) according to these embodiments comprises at least one magnetic strip (111), preferably arranged in the bottom section of the curtain (138).

The magnetic strip (111) is configured to interact with a corresponding portion (112) the lower support (11) of the disinfection device (10) having magnetic properties and/or with an additional member having magnetic properties. Herein the magnetic properties of the strip (111) and the corresponding portion (112) of the lower support (11) or the additional member are such that at least one of these element comprises a magnet, in particular a permanent magnet, whereas the other element(s) can be attracted by the magnet, e.g. because they comprise iron or another ferromagnetic material or are magnets themselves.

The magnetic strip provide weight to the curtain (110) which may help to ensure that the curtain falls back straight and vertical after being displaced by the item entering or leaving the tunnel (20) or the guides (32, 33). The magnetic connection between the curtain (110) and the lower support (11) or additional member provides a safe closed position, which further reduces UVC exposure of the personnel.

The magnetic strips (111) may also help to keep the respective pressures at either side of the tunnel when operating in an air lock from a room with lower purity grade to a room with higher purity grade such as a cleanroom, reducing pressure loss in the higher purity grade room.

In further embodiments of the invention that are not illustrated in the figures, the curtain comprises strips made of a relatively heavy material instead of the magnetic strips described above. These strips provide weight to the curtain (110) which may help to ensure the curtain falls back straight and vertical after being displaced by the item entering or leaving the tunnel (20) or the guides (32, 33).

In further embodiments of the invention that are not illustrated in the figures, the disinfection device (10) of any of the aforementioned embodiments further comprises a disinfectant application device for applying a disinfectant to the items (9) to be subjected to disinfection. The disinfectant may be applied to the items (9) prior to UVC irradiation and/or during UVC irradiation and/or after UVC irradiation. As disinfectant, for example at least one of hydrogen peroxide, in particular aerosolised hydrogen peroxide, peracetic acid and alcohol, in particular isopropanol and/or ethanol, may be used.

By combining the UVC irradiation technique with the treatment with a chemical disinfectant, a synergistic effect may be achieved, i.e. a particularly effective disinfection exceeding the single effects of UVC radiation or chemical treatment. By such combination, for example an effective disinfection may be possible even with relatively low doses of radiation and/or with relatively low amounts of chemical disinfectants.

In further embodiments of the invention that are not illustrated in the figures, the disinfection device (10) of any of the aforementioned embodiments further comprises a humidity control device adapted to maintain the relative humidity in at least a portion of the area in which the items are irradiated below a predefined threshold, wherein the threshold is 65 %, preferably 50 %, more preferably 40 %. As humidity control device, for example a dehumidifier such as an electric refrigeration dehumidifier may be used.

In further embodiments of the invention that are not illustrated in the figures, the disinfection device (10) of any of the aforementioned embodiments further comprises at least one optical magnification device placed between the radiation source (e.g. UVC lamp 21 or cassette 51) or one of multiple radiation sources and the items (9) to be subjected to disinfection treatment. As magnification device, a device acting as an optical lens may be used, for instance a spherical lens made of a material that is transparent to the UVC radiation such as quartz glass. By using such magnification device the irradiation efficiency may be increased by bundling the radiation.

In use, items (9) to be disinfected are inserted in the disinfection device (10) according to any embodiment of the invention. For this task, the items (9) may be manually or automatically inserted through the access slot (34) into the entry guard (32). The conveyor of the disinfection device (10) then transports the items from the entry guard (32) through the tunnel (20) into the exit guard (33). The items (9) may then be taken out of the exit guard (33) manually or automatically. In the tunnel, the items are irradiated with UVC radiation and thereby disinfected. Optionally, a disinfectant is applied the items (9) in the disinfection device (10) prior to and/or during and/or after the irradiation. If no entry guard (32) or exit guard (33) is present, the items (9) may for instance be directly inserted into the tunnel (20) or directly taken out of the tunnel (20).

By using the disinfection device (10) in this manner, the method of disinfecting items according to the invention may be carried out.

The efficacy of the disinfection device according to the present invention and the disinfection method according to the present invention was tested as follows. As no EN standards exist for the determination of efficacy of UVC light for disinfection purposes, the method of EN 13697 was adapted accordingly to obtain an appropriate and comparable testing method.

Samples were subjected to a disinfection treatment in accordance with the present invention, i.e. they were subjected to a disinfection treatment by UVC irradiation. The samples were exposed to different UVC doses by means of suitable settings (radiation power of the radiation sources, conveyor speed, etc.). Herein, a radiation having a wavelength of 254 nm was used. The distance between the radiation source and the samples was maintained at 20 cm during irradiation. After irradiation, the samples were treated as described in EN 13697 to determine surviving microorganism populations. Based on these results, the Log reductions were calculated. The Log reduction values obtained for the different doses are shown in the table below.

A 4 Log reduction in 5 minutes for bacteria and a 3 Log reduction in 15 minutes for fungal spores is considered acceptable according to EN 13697.

| Organism | 100 mJ/cm$^2$ | 150 mJ/cm$^2$ | 200 mJ/cm$^2$ | 200 mJ/cm$^2$ plus ethanol spray |
|---|---|---|---|---|
| *Bacillus Subtilus* Spores | 3.09 | 3.70 | 4.00 | 4.44 |
| *Staphylococcus aureus* | >6.01 | 5.53 | 5.74 | 5.62 |
| *Enterococcus hirae* | 3.28 | 3.62 | 4.07 | 4.80 |
| *E.coli* | 4.36 | 4.19 | 4.88 | 5.51 |
| *Pseudomonas aeruginosa* | 4.31 | 4.30 | 5.32 | 6.52 |
| *Candida albicans* | 4.03 | 4.20 | 4.79 | 6.25 |
| *Aspergillus brasiliensis* spores | 0.8 | 1.06 | 1.96 | 1.94 |

It is concluded from the Log reduction data in the table above that in accordance with the present invention there is a general trend that the Log reduction improves with the dose of UVC light applied. Slight variations in the measurement results may only occur because of typical experimental tolerances, e.g. because of differences in terms of DNA, RNA, proteins, spores, etc.

For all of the microorganisms except *Aspergillus brasiliensis* spores, an acceptable Log reduction was achieved, for most of them even at UVC doses of 100 mJ/cm$^2$ or 150 mJ/cm$^2$.

For *Aspergillus brasiliensis* spores, a continuous increase of the Log reduction was observed in line with UVC dose.

These results show that the disinfection device according to the present invention is a highly suitable and advantageous device for the intended purposes.

It is mentioned that the tests described above were performed with UVC radiation having a wavelength of 254 nm. Higher Log reductions may be expected for a higher UVC wavelength such as for instance 265 nm and/or higher irradiation intensities such as for instance up to 800 or up to 1500 mJ/cm$^2$.

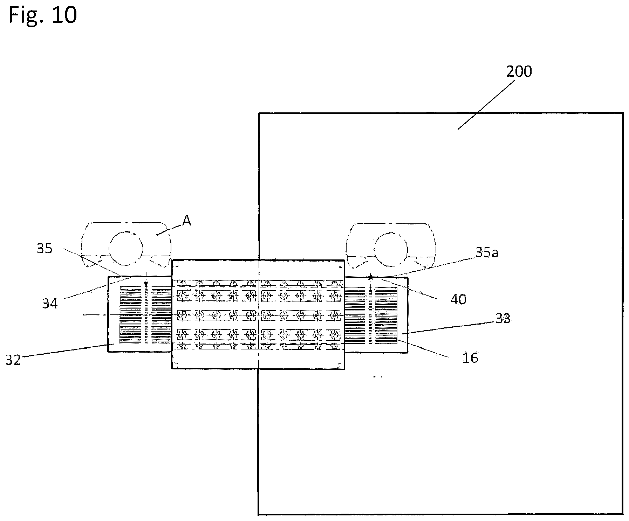

In Fig. 10, a system according to a further embodiment of the invention is shown. The system comprises a disinfection device (10) according to the invention and a cleanroom (200). Fig. 10 is shows the disinfection device (10) as illustrated in Fig. 2 (a) by way of example only. The disinfection device (10) being part of the system may be a disinfection device (10) according to any embodiment of the invention.

The entry guard (34) of the disinfection device (10) is located outside the cleanroom (200) whereas the exit guard (40) of the disinfection device (10) is located inside the cleanroom (200). Hence, by transporting items (9) through the disinfection device (10) and thereby disinfecting these items (9), these items are at the same time introduced in the cleanroom (200).

By using the system in this manner, the method of introducing items into a cleanroom according to the invention may be carried out.

**Claims**

1. A disinfection device comprising

   - a conveyor for conveying items to be subjected to disinfection treatment in a forward direction,
   - an irradiation device for irradiating the items to be subjected to disinfection treatment during their transport by the conveyor,
   wherein the irradiation device comprises at least one radiation source for emitting a UVC radiation, and
   - a guiding device for guiding the items to be subjected to disinfection treatment while being conveyed in a direction that is other than the forward direction.

2. The disinfection device according to the preceding claim,

   wherein the guiding device is oriented in a direction that is inclined relative to the forward direction both vertically and horizontally, and/or
   wherein the guiding device comprises a nudge wire and/or a nudge bar.

3. The disinfection device according to any of the preceding claims,

   wherein the conveyor comprises a conveyor belt partially transparent to the UVC radiation, in particular a mesh conveyor belt, especially a wire mesh conveyor belt,
   wherein the irradiation device comprises at least one radiation source arranged underneath the conveyor, and
   wherein the guiding device is configured to move the items to be subjected to disinfection treatment across the forward direction, in particular in a sliding movement, on the conveyor belt,
   wherein preferably the conveyor belt comprises a belt body and suspensions protruding from the belt body for suspending the items to be subjected to disinfection above the surface of the belt body, and/or
   wherein preferably the mesh belt comprises mesh openings with a total area of at least 50 %, in particular at least 75 % of the total surface area of the conveyor belt.

4. The disinfection device according to any of the preceding claims,

   wherein the radiation source is tunable with respect to the wavelength of the emitted radiation, in particular by means of a controller, and/or
   wherein the irradiation device source comprises a semiconductor radiation source,
   wherein preferably the irradiation device comprises at least one LED,
   wherein more preferably the at least one radiation source is an LED or a plurality of LEDs.

5. The disinfection device according to any of the preceding claims,

   wherein the UVC radiation has a wavelength of at least 150 nm and/or a wavelength of at most 280 nm,
   wherein preferably the UVC radiation has a wavelength of at least 200 nm and/or a wavelength of at most 280 nm,
   wherein more preferably the UVC radiation has a wavelength of at least 260 nm and/or a wavelength of at most 280 nm.

6. The disinfection device according to any of the preceding claims,

   further comprising a disinfectant application device for applying a disinfectant to the items to be subjected to disinfection,
   wherein the disinfection device is adapted to apply the disinfectant to the items to be subjected to disinfection prior to and/or during and/or after the irradiation with the UVC radiation,

wherein preferably the disinfectant is at least one of

- hydrogen peroxide, in particular aerosolised hydrogen peroxide,
- peracetic acid,
- alcohol or an alcohol water mixture, in particular isopropanol and/or ethanol or a mixture of water with isopropanol and/or ethanol,

and/or

wherein preferably the disinfectant application device comprises a chamber for accommodating the items during the application of the disinfectant.

7. The disinfection device according to any of the preceding claims,

further comprising a gas flow device for applying a gas flow to the items to be subjected to disinfection treatment,
wherein preferably the gas flow device is a gas blade device, and/or
wherein preferably the gas flow is an air flow, more preferably a flow of purified air, most preferably a flow of disinfected air.

8. The disinfection device according to any of the preceding claims,

wherein the irradiation device is adjustable with respect to the horizontal distance between the radiation source or at least a part of multiple radiation sources and the items to be subjected to disinfection treatment, wherein the horizontal distance is measured in a direction perpendicular to the direction of the forward direction and the vertical direction,
wherein preferably the irradiation device is in addition adjustable with respect to the vertical distance between the radiation source or at least a part of multiple radiation sources and the items to be subjected to disinfection treatment, and/or
wherein preferably the disinfection device further comprises a movement device for moving the radiation source or at least a part of multiple radiation sources and thereby adjust the horizontal distance and/or the vertical distance,
wherein more preferably the movement device comprises an actuator controlled by an electrical selector switch and/or a control unit,
wherein in particular the actuator is an electric stepper motor, an electric linear actuator, a pneumatic actuator, or a hydraulic actuator.

9. The disinfection device according to any of the preceding claims,

further comprising a tunnel with an entry orifice and an exit orifice,
wherein the conveyor is adapted to convey the items to be subjected to disinfection treatment from the entry orifice to the exit orifice,
wherein the UVC radiation is emitted in the interior of the tunnel,
wherein preferably the tunnel comprises internal surfaces that are reflective to the UVC radiation, and/or
wherein preferably the disinfection device further comprises a tunnel and the gas flow device is configured to generate a gas flow flowing in the direction from the exit orifice to the entry orifice.

10. The disinfection device according to any of the preceding claims,
comprising at least four radiation sources whereof

- at least two are arranged at either side of the conveyor with respect to the direction of conveyor movement to horizontally irradiate the items to be subjected to disinfection treatment,
- at least one is arranged above the conveyor to vertically irradiate the items to be subjected to disinfection treatment from above, and
- at least one is arranged underneath the conveyor to vertically irradiate the items to be subjected to disinfection treatment from below, the conveyor being at least partially transparent to the UVC radiation.

11. The disinfection device according to any of the preceding claims,

further comprising an image and/or size recognition device including a camera and/or a sensor,

wherein the disinfection device is configured to be adjusted according to an image or multiple images of and/or a size information about the items to be subjected to disinfection,

wherein preferably adjusting the disinfection device includes adjusting at least one of the following properties:

- horizontal distance between the radiation source or at least a part of multiple radiation sources and the items to be subjected to disinfection treatment,
- vertical distance between the radiation source or at least a part of multiple radiation sources and the items to be subjected to disinfection treatment,
- conveyor speed,
- UVC emission power of the radiation source or at least a part of multiple radiation sources.

12. Use of the disinfection device according to any of the preceding claims as a transport connection between a first room and a second room,

wherein items are transportable from the first room into the second room through the disinfection device, so that the items are irradiated with the UVC radiation during their transport,
wherein preferably the first room and the second room are separated from each other apart from the connection through the disinfection device.

13. System comprising

- a cleanroom and
- a disinfection device according to any of claims 1 to 18,

wherein the disinfection device is configured and/or positioned to subject items to be introduced in the cleanroom to a disinfection treatment.

14. Method of disinfecting items comprising the steps of

- transporting the items in a forward direction using a conveyor, preferably transporting the items within a tunnel from an entry orifice to an exit orifice,
- irradiating the items with UVC radiation while being transported, and
- guiding the items to be subjected to disinfection treatment while being conveyed in a direction that is other than the forward direction.

15. Method of introducing items into a cleanroom comprising the steps of

- transporting the items in a forward direction from outside the cleanroom into the cleanroom using a conveyor, preferably transporting the items within a tunnel from an entry orifice communicating with the outside of the cleanroom to an exit orifice communicating with the interior of the cleanroom,
- irradiating the items with UVC radiation while being transported, and
- guiding the items to be subjected to disinfection treatment while being conveyed in a direction that is other than the forward direction.

Fig. 1

Fig. 2

(a)

(b)

(c)

Fig. 3

(a)                                              (b)

Fig. 4

## Fig. 5

# Fig. 6

(a)

16

80

84

32

9

20

80

11

88

21

85

(b)

Fig. 7

Fig. 8

100          101

Fig. 9

Fig. 10

Europäisches
Patentamt

European
Patent Office

Office européen
des brevets

## EUROPEAN SEARCH REPORT

**Application Number**

EP 21 21 4112

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 113 332 467 A (WANG WEI) 3 September 2021 (2021-09-03) | 1,2,4-6, 9,12-14 | INV. A61L2/10 |
| A | * Figures: 1, 2 Paragraphs: 0027, 0042, 0036, 0010, 0011, 0006 * | 3 | A61L2/16 A61L2/18 A61L2/26 B08B9/30 |
| X | CN 208 998 497 U (SICHUAN XIULINGCHUNTIAN DEV CO LTD) 18 June 2019 (2019-06-18) | 1,2,4,7, 9,14 | B08B9/42 |
| A | * Figures: 1 Paragraphs: 0015, 0013, 0035 * | 3 | |
| X | CN 212 397 571 U (GUANGDONG BAIWEIJIAWEI TASTE INDUSTRY TECH CO LTD) 26 January 2021 (2021-01-26) | 1,2,4,7, 14 | |
| A | * Figures: 1, 3 Paragraphs: 0010, 0026, 0034 * | 3 | |
| X | CN 211 068 197 U (GANSU YUBAOZE AGRICULTURAL RECLAMATION POPPYSEED BIOLOGICAL DEV CO LTD) 24 July 2020 (2020-07-24) | 1,2,4,14 | |
| A | * Figures: 1 Paragraphs: 0036 * | 3 | **TECHNICAL FIELDS SEARCHED (IPC)** A61L B08B |
| X | US 2020/297004 A1 (ALZEER MOHAMMED [SA] ET AL) 24 September 2020 (2020-09-24) | 10 | |
| A | * Figures: 1 Paragraphs: 0005, 0032, 0008 * | 3 | |

The present search report has been drawn up for all claims

| Place of search | Date of completion of the search | Examiner |
|---|---|---|
| The Hague | 19 May 2022 | Accettola, Francesca |

EPO FORM 1503 03.82 (P04C01)

Europäisches
Patentamt

European
Patent Office

Office européen
des brevets

**Application Number**

**EP 21 21 4112**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**2-7, 9, 10, 12-15(completely); 1(partially)**

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

    1. claims: 2-7, 9, 10, 12-15(completely); 1(partially)

        A disinfection device comprising a conveyor, a UV emitting
        device and a guiding device, wherein the conveyor is
        partially transparent to UV radiation, the UV emitting
        device arranged underneath the belt conveyor and the guiding
        device allows the item to be disinfected to slide across the
        forward.
                            ---


    2. claims: 8(completely); 1(partially)

        A disinfection device comprising a conveyor, a UV emitting
        device and a guiding device, wherein the horizontal and
        vertical distances of the irradiation device from the items
        to be disinfected is adjustable
                            ---


    3. claims: 11(completely); 1(partially)

        A disinfection device comprising a conveyor, a UV emitting
        device and a guiding device, wherein the device comprises an
        image and/or size recognition device including a camera
        and/or a sensor, and wherein the disinfection device is
        configured to be adjusted according to an image or multiple
        images of and/or a size information about the items to be
        subjected to disinfection.
                            ---

## ANNEX TO THE EUROPEAN SEARCH REPORT
## ON EUROPEAN PATENT APPLICATION NO.

EP 21 21 4112

This annex lists the patent family members relating to the patent documents cited in the above-mentioned European search report.
The members are as contained in the European Patent Office EDP file on
The European Patent Office is in no way liable for these particulars which are merely given for the purpose of information.

19-05-2022

| Patent document cited in search report | | Publication date | Patent family member(s) | Publication date |
|---|---|---|---|---|
| CN 113332467 | A | 03-09-2021 | NONE | |
| CN 208998497 | U | 18-06-2019 | NONE | |
| CN 212397571 | U | 26-01-2021 | NONE | |
| CN 211068197 | U | 24-07-2020 | NONE | |
| US 2020297004 | A1 | 24-09-2020 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **A. DOWNES et al.** *Proceedings of the Royal Society of London,* vol. 28 (190-195), 199-212

- *Zeitschrift für Allgemeine Physiologie,* 1904, vol. 4 (1-43), 5, , 95-122, 105